# EUROPÄISCHE PATENTSCHRIFT

(11) **EP 2 857 050 B1**
(45) Veröffentlichungstag und Bekanntmachung des Hinweises auf die Patenterteilung: **28.12.2016**
(21) Anmeldenummer: 14199403.8
(22) Anmeldetag: 26.08.2003
(51) Int. Cl.: A61L 29/16, A61L 29/08, A61M 25/10

(54) **Medizinische Vorrichtung zur Arzneimittelabgabe**
Medical device for delivering drugs
Dispositif médical d'administration de médicament

(30) Priorität: 20.09.2002 DE 10244847
(43) Veröffentlichungstag der Anmeldung: 08.04.2015
(62) Teilanmeldung aus: 07017424.8
(73) Patentinhaber: Bayer Intellectual Property GmbH, 40789 Monheim (DE)
(72) Erfinder: Speck, Prof. Dr. Ulrich, 13465 Berlin (DE); Scheller, Dr. Bruno, 66111 Saarbrücken (DE)
(74) Vertreter: Zimmermann & Partner Patentanwälte mbB

(56) Entgegenhaltungen:
- WO-A1-01/49268
- WO-A1-01/52772

## Beschreibung

einen Ballonkatheter und ein Verfahren zur Beschichtung eines Ballonkatheters. Die Erfindung betrifft Zahlreiche Erkrankungen betreffen nicht den gesamten Organismus gleichzeltig sondern sind auf bestimmte Gewebearten, häufig auch auf sehr begrenzte einzelne Gewebebezirke oder Organteile beschränkt. Beispiele dafür finden sich unter den Tumor-, Gelenk- und Gefäßerkrankungen.

Die Pharmakotherapie auch dieser Erkrankungen erfolgt im allgemeinen durch orale oder intravenöse Gabe von Arzneistoffen, die sich im ganzen Körper verteilen und in vielen Fällen gerade bei schweren Erkrankungen unerwünschte Wirkungen in gesunden Geweben und Organen verursachen, die die therapeutische Anwendung begrenzen. Eine selektive Therapie der erkrankten Gewebe wurde mittels spezifisch an erkranktes Gewebe bindende Arzneistoffen (z. B. Antikörper) unter Beibeihaltung des Applikationsweges oder durch selektive Verabreichung, z.B. durch direkte Injektion in das erkrankte Gewebe oder durch Zufuhr über Katheter zu den das erkrankte Gewebe versorgenden Blutgefäßen, erreicht. Im Falle der selektiven Verabreichung entstehen durch die meist kurze Wirkdauer der Arzneistoffe und die invasive Verabreichungswege Probleme, da sich eine beliebig wiederholte Gabe verbietet. Bei selektiver Verabreichung über den das erkrankte Gewebe versorgenden Blutstrom ergibt sich das zusätzliche Problem ungenügender Extraktion der Arzneistoffe bei der raschen Passage des Blutes oder der Wirkstofflösung durch die Blutgefäße.

Diesen Problemen wurde bisher durch unterschiedliche pharmazeutische Präparate mit verzögerter Wirkstofffreigabe, arzneimittelfreisetzende Implantate oder für längere Zeit funktionsfähige selektive Zugangswege wie implantierte Katheter etc. begegnet.

Es ist bereits bekannt, die Oberfläche von in den Körper eingebrachten medizinischen Geräten, insbesondere Kathetern, mit Mitteln zur Verbesserung der Gleitfähigkeit oder zur Verhinderung der Blutgerinnung, jedoch ohne therapeutische Wirkung, zu beschichten.

Darüber hinaus werden Katheter mit speziellen Vorrichtungen versehen, um Arzneimittel in die Arterienwand zu injizieren, beispielsweise mittels Nadeln oder hohem Injektionsdruck über eine an der Gefäßwand anliegende Perforation der Katheterwand.

Andere Prinzipien beruhen darauf, die Kontaktzeit zwischen Arterienwand und einer über den Katheter applizierten Wirkstoffzubereitung zu verlängern, indem entweder der Blutstrom für einen entsprechenden Zeitraum unterbunden wird, z. B. Doppelballonkatheter mit zwischen den Ballons befindlicher mit der Arzneistofflösung gefüllte Kammer oder Hohlräumen zwischen der z.B. mit Wülsten versehenen Ballonaußenwand, wobei der Blutstrom durch einen den Ballon passierenden Kanal in begrenztem Maße aufrechterhalten werden kann.

Gemäß der US 5 102 402 werden Arzneistoffe in Form von Mikrokapseln zur verzögerten Wirkstofffreigabe lose in vorgeformten Vertiefungen von Ballonkathetern untergebracht. Nach Expansion des Ballons sollen die Mikrokapseln in die Gefäßwand gedrückt werden, dort verbleiben und den oder die Wirkstoffe langsam freisetzen. Zahlreiche Autoren schlagen auch vor, Arzneistoffe in Hydrogel eingebettet auf Ballonkatheter aufzubringen, wobei die Funktion des Hydrogels als Haftmittel, zur Verbesserung der Gleitfähigkeit oder Verzögerung der Freisetzung der Arzneistoffe offen bleibt.

Nachteil der genannten Produkte ist in jedem Falle der komplexe Aufbau mit entsprechenden Problemen bei Herstellung, Qualitätskontrolle und Kosten sowie zusätzlichen, Arzt und Patient belastenden Arbeitsschritten bei der Anwendung. Ein Teil der genannten Methoden führt zu einer über die beabsichtigte Gefäßerweiterung hinausgehenden gänzlich unerwünschten Gefäßverletzung. Andererseits hat jede zur Verlängerung der Kontaktzeit vorgesehene Maßnahme eine zusätzliche Minderversorgung der nachgeschalteten Gewebe mit Blut und Sauerstoff zur Folge.

Der Vollständigkeit halber wird noch auf eine in der WO 01/24866 beschriebenen Vorrichtung zum Verhindern der Restenose verwiesen, die mit einer von natürlichen Zellmembranen abgeleiteten lipiden Ceramide-Substanz beschichtet sind. Diese Substanz wird aufgrund ihrer bei gebräuchlichen Arzneistoffen nicht anzutreffenden Affinität zu den Zellwänden der Arterienwand verwendet. In der Fachliteratur wird jedoch weiterhin die Auffassung vertreten, dass eine Arzneimittelprophylaxe der Restenose eine Freisetzung der erforderlichen Wirkstoffe über Tage erfordert.

Dokument WO 01/49268 A1 betrifft pharmazeutische Formulierungen für die Zuführung von Arzneimitteln, welche eine geringe Wasserlöslichkeit aufweisen.

Der Erfindung liegt die Aufgabe zugrunde, eine Vorrichtung für eine auf bestimmte Gewebebereiche oder Organteile begrenzte Arzneimittelabgabe bereitzustellen, die ohne schädigenden Einfluss auf gesundes Gewebe eine starke therapeutische Wirkung ausübt, den Patienten nur wenig belastet und mit geringem Aufwand angewendet und hergestellt werden kann.

Erfindungsgemäß wird die Aufgabe mit einer gemäß den Merkmalen der Ansprüche 1, 10 und 15 ausgebildeten bzw. hergestellten Vorrichtung gelöst. Aus den Unteransprüchen ergeben sich weitere Merkmale und vorteilhafte Weiterbildungen der Erfindung.

Durch die Erfindung werden in einem einfachen Herstellungsverfahren verbesserte arzneistofftragende Ballonkatheter bereitgestellt, die vielseitig anwendbar sind und eine sofortige Wirkstofffreigabe ermöglichen. Überraschend und entgegen der Lehrmeinung ist keine andauernde Wirkstofffreisetzung aus einer inerten Matrix (Polymer,Hydrogel, Mikrokapseln etc.) oder speziellen chemischen oder physikalischen Zuständen der Wirkstoffe erforderüch oder nützlich. Daher werden auch keine aufwendigen Techniken zur Produktion oder Kontrolle von Depotforntulierungen benötigt.

Die Beschichtung von auf Kathetern befindlichen Ballonen mit Arzneistoffen gemäß der vorliegenden Erfindung ist insofern von besonderem Nutzen als nach dem Erweitern von Blutgefäßen oder anderen Hohlräumen im Körper mit den Ballonen häufig der Bedarf an therapeutischen Maßnahmen besteht, um eine Verengung oder einen Verschlusa des mit dem Ballon unter Druck geschaffenen Lumens zu verhindern, Tumorwachstum zu begrenzen oder Heilungsprozesse einschließlich der Bildung von Kollateralkreisläufen zu fördern. Dies kann durch Arzneistoffe erreicht werden, die ihre Wirkung in unmittelbarer Nähe der Ballonoberfläche entfalten. Die Arzneistoffe haften auf dem Weg zum Ziel - meist durch intensiv durchblutete Arterien - bis zur Entfaltung des Ballons fest auf diesem, werden dann während der kurzen, oft nur Sekunden andauernden Kontaktzeit des entfalteten Ballons an das Gewebe in wirksamer Dosis abgegeben und von diesem in einer Weise aufgenommen, die das Abspülen durch den nach Deflation des Ballons sofort wieder einsetzenden Blutstrom vermeidet.

Zur Beschichtung sind erfindungsgemäß Katheter bzw. Teile von Kathetern, die zumindest für kurze Zeit mit Druck gegen erkrankte Gewebe gepresst werden, vorgesehen. Bevorzugte Kathetermaterialien sind Polyamide, Polyamid-Gemische und Copolymere, Polyethylenterephthalat, Polyethylen und Copolymere, Polyurethan, Naturkautschuk und dessen Derivate. Die Länge und der Durchmesser der für die Pharmakotherapie vorgesehenen Bereiche der Katheter bzw. Ballone ist für die Anwendung nicht von entscheidender Bedeutung, da die Dosierung in µg Wirkstoff/ mm² Oberfläche berechnet wird. Beispielsweise sind für die Koronardilatationen Ballone im Bereich von 2 - 4 mm Durchmesser und von 1,0-4,0 cm Länge gebräuchlich. Für andere Gefäße können auch Ballone bis zu > 20 mm Durchmesser und Längen bis zu > 10 cm eingesetzt werden. Die zu beschichtenden Oberflächen können glatt (d.h. ohne besondere Struktur zur Aufnahme der Wirkstoffe), aufgeraut oder in beliebiger Weise mit Strukturen versehen sein, wobei spezielle oberflächenstrukturen nicht Voraussetzung für die Haftung der Wirkstoffe sind, die Haftung aber auch nicht behindern. Die Haftung der Wirkstoffe auf den Ballonoberflächen wird ausschließlich durch die Wahl geeigneter Lösungsmittel und ggf. die Haftung beeinflussende Zusatzstoffe bewirkt. Sie ist selbst auf äußerlich vollständig glatten Ballonoberflächen überraschend fest.

Alle Oberflächen können zusätzlich mit Substanzen beschichtet worden sein oder werden, die die Gleitfähigkeit der Produkte verbessern, die Gerinnung von Blut an der Oberfläche verhindern oder sonstige Eigenschaften der Medizinprodukte verbessern, ohne dass die zur Beschichtung benutzten Materialien an die Umgebung abgegeben werden müssen und ohne dass die Beschichtung die Abgabe der Wirkstoffe zur Behandlung der Zielgewebe und damit die Wirksamkeit wesentlich einschränkt.

Ballonkatheter werden aus sehr dünnen Kunststoffschläuchen durch Aufweitung eine Segments von 1 bis ca. 10 cm Länge geformt. Die aufgeweitete, sehr dünnwandige Ballonmembran wird anschließend in mehrere längs zur Katheterachse angeordnete Falten gelegt und fest um die Katheterachse gewickelt, so dass der aufgeweitete Bereich im gefalteten Zustand einen nur minimal größeren Durchmesser aufweist als der übrige Katheter. Die enge Faltung der Ballonhülle ist Voraussetzung für die problemlose Passage des Ballonkatheters durch Einführungsschleusen, Führungskatheter und z.B. stark verengte Abschnitte von Blutgefäßen.

Die Ballone von Kathetern können in gefaltetem und entfaltetem Zustand beschichtet werden, wobei in jedem Fall eine intakte, ausreichend gleichmäßige Beschichtung der Oberfläche erzielt wird und die Wirkstoffe auch während des Einfaltens eines im entfalteten Zustand beschichteten Ballonkatheters ausreichend fest an dessen Oberfläche haften.

Die Herstellung eines in entfaltetem Zustand beschichteten Ballons erfolgt ohne Beeinträchtigung der Beschichtung beispielsweise durch die Verwendung von Ballonhüllen mit vorgeformten Falten und Biegungen, deren Struktur im Material, durch das Aufdehnen nicht verloren geht und die nach Ablassen des Druckes aus dem Ballon bewirken, dass sich die Ballonhülle wieder regelrecht wenigstens lose einfaltet, ohne dass es einer äußeren Kraft als primäre Ursache bedarf. Erst danach werden die vorgeformten Falten von außen oder durch Vakuum zusammengepresst. In keinem Falle sind Falten zum Festhalten des Wirkstoffs erforderlich. Weiterhin kann die Einfaltung durch geringe mechanische Kräfte mittels sehr glatter Materialien bewirkt werden, wobei die Werkzeuge auch beispielsweise mit schlüpfrigen biokompatiblen Flüssigkeiten benetzt sein können, in denen sich die Wirkstoffe nicht oder jedenfalls nicht gut lösen.

Gemäß einer weiteren Erfindungsvariante werden die Ballone fertig gefalteter Ballonkatheter durch Tauchen in niedrig viskose Wirkstofflösungen beschichtet. Dabei dringen Lösungsmittel und Wirkstoff zwischen die extrem engen Falten und bilden dort einen überraschend gleichmäßigen und in der Dosis reproduzierbaren Belag, der durch keinen weiteren Arbeitsschritt beschädigt wird. Die außen anhaftende Lösung bzw. der nach Trocknen des Lösungsmittels außen anhaftende Belag kann dort belassen oder in einem weiteren Arbeitsschritt entfernt werden, so dass nur der von den Falten des Ballons verdeckte Wirkstoff erhalten bleibt.

Nach der Beschichtung kann bei gefaltetem Ballon ein Stent auf den Ballonkatheter geschoben und auf diesem festgepresst werden. Danach ist nur noch die Sterilisation, z. B. mittels Ethylenoxid, erforderlich.

Der so gestaltete Arbeitsgang ist außerordentlich einfach, wenig störanfällig und auch mit mechanisch, chemisch und physikatisch empfindlichen Beschichtungsrnaterialien durchzuführen. Es hat sich gezeigt, dass die Beschichtung nach diesem Verfahren zu keiner unerwünschten Lockerung oder Verklebung der Faltung führt und dass der derart aufgetragene Wirkstoff fest genug haftet, um auf dem Weg durch das Blut nicht abgetragen zu werden, andererseits bei Inflation des Ballons im Zielgewebe den Wirkstoff weitgehend freigibt.

Als Arzneistoffe kommen stark lipophile, weitgehend wasserunlösliche, an beliebige Gewebebestandteile bindende stark wirksame Arzneistoffe in Frage. Als lipophil werden Arzneistoffe bezeichnet, deren Verteilungskoeffizient Butanol: wässriger Puffer pH 7 = 0.5, bevorzugt = 1 und besonders bevorzugt = 5 bzw. Octanol: wässriger Puffer pH 7 = 1, bevorzugt = 10, besonders bevorzugt > 50 ist. Alternativ oder zusätzlich sollen die Arzneistoffe zu > 10 %, bevorzugt zu > 50 %, besonders bevorzugt zu > 80 % reversibel und/oder irreversibel an Zellbestandteile binden. Erfindungsgemäß werden Substanzen zur Hemmung der Zellproliferation oder auch entzündlicher Prozesse oder Antioxidantien wie Paclitaxel und andere Taxane, Rapamycin und verwandte Substanzen, Tacrolimus und verwandte Substanzen, Corticoide, Sexualhormone (Östrogen, Estradiol, Antiandrogene) und verwandte Substanzen, Statine, Epothilone, Probucol, Prostacycline, Angiogeneseinduktoren eingesetzt.

Weiterhin hierin beschrieben ist, dass die Substanzen als trockene Festsubstanz oder als öl auf den, Oberflächen der unterschiedlichen Medizinprodukte vorliegen konnen. Dabei bevorzugt sind Partikel geringster Größe (in der Mehrzahl < 5 µm, bevorzugt < 1 µm, besonders bevorzugt < 0.1 µm), besonders bevorzugt sind amorphe, nicht kristalline Strukturen feinster Partikelgröße, die bei Kontakt mit Gewebe wegen ihrer großen Oberfläche trotz grundsätzlich geringer Wasserlöslichkeit der Arzneistoffe rasch in Lösung gehen und nicht als Mikrokapseln fungieren, d. h. sich spontan und schnell lösen. Dabei ist es ausreichend, dass eine wirksame Dosis in Form kleinster oder amorpher Partikel vorliegt; größere Partikel tragen zwar zur Wirkstoffkonzentration im Gewebe kaum bei, stören aber auch nicht. Die Dosierung richtet sich nach der erwünschten Wirkung und der Wirksamkeit des verwendeten Arzneistoffes. Sie kann bis zu 5 µg/mm² erreichen, wobei dies keine Obergrenze darstellt. Geringere Dosierungen sind leichter zu realisierten.

Eine gute Haftung an den Oberflächen der Katheter, Nadeln oder Drähte bei Verbesserung der Aufnahme in die Gewebe wird durch Einbetten von stark lipophilen, schlecht wasserlöslichen Wirkstoffen in eine leicht wasserlösliche Matrixsubstanz erreicht. Als Matrixsubstanzen sind niedermoleklare (Molekulargewicht < 5000 D, bevorzugt < 2000 D) hydrophile Substanzen wie in vivo verwendete Kontrastmittel und Farbstoffe für unterschiedliche diagnostische Verfahren in der Medizin, Zucker und verwandte Substanzen wie Zuckeralkohole, niedermolekulare Polyethylenglycole, biokompatible organische und anorganische Salze wie z. B. Benzoate, Salze und andere Derivate der Salizylsäure etc. geeignet. Als Kontrastmittel sei auf die jodierten Röntgenkontrastmittel und die paramagnetischen Chelate verwiesen, Beispiele für Farbstoffe sind Indocyaningrün, ipluorescoin und Methylenblau. Hilfsstoffe können auch zur Verbesserung der Lagerfähigkeit der Produkte dienen, spezielle ergänzende pharmakologische Wirkungen verursachen oder der Qualitätskontrolle dienen.

Erfindungsgemäß wird als Matrixsubstanz ein Zuckeralkohol eingesetzt.

Hilfsstoffe aller Art können in geringerer oder höherer Dosis als die Wirkstoffe eingesetzt werden.

Die Beschichtung der Medizinprödukte erfolgt mittels Lösungen, Suspensionen oder Emulsionen der genannten Arzneistoffe und Hilfsstoffe. Geeignete Lösungs- Suspendieroder Emulsionsmedien sind beispielsweise Ethanol, Isopropanol, Ethylacetat, Diethylether, Aceton, Dimethylsulfoxid, Dimethylformamid, Glycerin, Wasser oder Mischungen derselben. Die Auswahl der Lösungsmittel folgt entsprechend der Löslichkeit der Wirkstoffe und Zusatzstoffe sowie der Benetzung der zu beschichtenden Oberflächen und der Wirkung auf die Struktur der nach Verdampfen der Lösungsmittel zurückbleibenden Beschichtung und Partikel, deren Haftung auf der Oberfläche und die Wirkstoffübertragung in das Gewebe während sehr kurzer Kontaktzeiten.

Die Auftragung kann beispielsweise durch Tauchen, Bestreichen, Auftragen mittels Volumenmesseinrichtungen oder Besprühen jeweils bei unterschiedlichen Temperaturen und ggf. Dampfsättigungen der Lösungsmittel in der Atmosphäre geschehen. Der Vorgang kann mehrfach, ggf. auch unter Verwendung verschiedener Lösungsmittel und Hilfsstoffe, wiederholt werden.

Die Ballone fertig gefalteter Ballonkatheter lassen sich durch Tauchen in wirkstoffhaltige Lösungen oder andere Maßnahmen erstaunlich gleichmaßig, reproduzierbar, in der Dosis steuerbar und ohne Beeinträchtigung der Funktion der Katheter beschichten. Bei wiederholtem Tauchen in ungesättigten Wirkstoffläsurigen kommt es wider Erwarten nicht zu einer vollständigen Ablösung des vorher aufgetragenen Wirkstoffs sondern zu einer reproduzierbaren Erhöhung des Wirkstoffgehaltes der Ballone.

Überschüssige Lösung bzw. überschüssige, außen lose anhaftende Substanzen aus der Beschichtungslösung können mit einfachen Methoden entfernt werden, ohne dass es zu einer Beeinträchtigung der Wirksamkeit der Beschichtung kommt.

Die erfindungsgemäß ausgebildeten und hergestellten medizinischen Vorrichtungen unterschiedlicher Art kommen kurzzeitig, d. h. für Sekunden, Minuten oder wenige Stunden mit dem Gewebe in Kontakt. In einigen Fällen ist es erwünscht, das Gewebe in unmittelbare Nähe des Medizinproduktes pharmakologisch zu behandeln, zum Beispiel an überschießendem Wachstum als Reaktion auf eine Versetzung zu hindern oder Tumorwachstum zu reduzieren oder die Einsprossung von Blutgefäßen zu fördern oder Entzündungsreaktionen zu vermindern. In all diesen Fällen kann mit dem oben beschriebenen Verfahren eine hohe lokale Arzneistoffkonzentration für erstaunlich lange Zeit erzielt werden. Ein wesentlicher Vorteil ist die außerordentliche Vielfalt der Einsatzmöglichkeiten der beschriebenen Produkte und Verfahren.

Eine bevorzugte Anwendung ist die Verminderung der durch die Gefäßdilatation mittels Ballonkathetern induzierte Hyperproliferation der Gefäßwände. Diese ist im Bereich gegebenenfalls inplantierter Gefäßstützen (Stents) auch durch Beschichtung der Stents mit Arzneistoffen zu erzielen, allerdings, nur im unmittelbar durch den Stent bedeckten Gefäßbereich. Die beschichteten Ballonkatheter behandeln darüber hinaus die behandlungsbedürftigen Bereiche kurz vor und kurz hinter dem Stent, sie können ohne erneute Stentimplantation den Bereich innerhalb bereits vorhandener Stents behandeln oder Gefäße, in die kein Stent implantiert werden soll oder kann. Vorteilhaft gegenüber den über einen langen Zeitraum Arzneistoff freisetzenden Stents ist die bessere Einteilung bei gleichzeltig guter Hemmung der Hyperproliferation und das geringere Thromboserisiko.

Nachfolgend werden mehrere Beispiele der Beschichtung von Ballonkathetern sowie die Haftung der Beschichtung im Blut, die Restenosehemmung und den Wirkstoffgehalt der Katheter beschrieben.

### Vergleichsbeispiel 1:

Beschichten eines expandierten Ballonkatheters mit Paclitaxel in Ethylacetat

Ballonkatheter der Fa. BMT, Oberpfaffenhofen/ München, Deutschland, mit der Bezeichnung Joker Lite, Ballongröße 2.5 mm x 20 mm, werden nach maximaler Expansion für 1 min. über die gesamte Ballonlänge in Ethylacetat, 18.8 mg Paclitaxel/ ml, + 1% pharmazeutisches Olivenöl, getaucht, getrocknet:
Paclitaxelgehalt 39 *µ*g (nach Extraktion mit Ethanol, HPLC).

### Vergleichsbeispiel 2:

Beschichten eines gefalteten Ballonkatheters mit Paclitaxel in Ethylacetat Ballonkatheter der Fa. BMT, Oberpfaffenhofen/ München, Deutschland, mit der Bezeichnung Joker Lite, Ballongröße 2.5 mm x 20 mm, werden im gefalteten Zustand für 1 min. über die gesamte Ballonlänge in Ethylacetat, 18.8 mg Paclitaxel/m1, + 1% pharmazeutisches Olivenöl, getaucht, getrocknet:
Paclitaxelgehalt 69 *µ*g.

### Vergleichsbeispiel 3

:
Beschichten eines gefalteten Ballonkatheters mit Paclitaxel in Ethylacetat
   a) Ballonkatheter der Fa. BMT, Oberpfaffenhofen/ München, Deutschland mit der Bezeichnung Joker Lite, Ballongröße 2.5 mm x 20 mm, werden im gefalteten Zustand für 1 min. über die gesamte Ballonlänge in Ethylacetat, 16.6 mg Paclitaxel/ ml, getaucht, 4 h getrocknet: Paclitaxelgehalt 54 *µ*g
b) Ebenso, jedoch noch 2 mal 5 sec. mit 1 h Trocknungszeit nach jedem Tauchvorgang in Lösung A (= 3.33 ml Ethylacetat + 100.0 mg Paclitaxel) getaucht: Paclitaxelgehalt 126 *µ*g
c) Ebenso, jedoch noch 4 mal 5 sec. mit 1 h Trocknungszeit nach jedem Tauchvorgang in die gleiche Lösung getaucht: Paclitaxelgehalt 158 *µ*g

### Vergleichsbeispiel 4:

Beschichten eines Ballonkatheters mit Paclitaxel in Aceton 350 mg Paclitaxel in 9.0 ml Aceton lösen; Ballonkatheter der Fa. BMT, Oberpfaffenhofen/ München, Deutschland mit der Bezeichnung Joker Lite, Ballongröße 2.5 mm x 20 mm, werden im maximal expandierten Zustand für 1 min. über die gesamte Ballonlänge getaucht, entnommen, das Lösungsmittel wird bei Raumtemperatur 12 h getrocknet. Danach wird der Ballon deflatiert und mit PTFE-beschichtetem Werkzeug in üblicher Weise eingefaltet. Optional kann ein Stent geeigneter Größe auf den Ballon gecrimmt werden: 29 *µ*g Paclitaxel auf dem Ballon.

### Beispiel 1 :

Beschichten eines Ballonkatheters mit Paclitaxel in Aceton
   a) Tauchen gefaltete Ballonkatheter der Fa. BMT mit der Bezeichnung Allegro, Ballongröße 2.5 x 20 mm in ein Gemisch von 0.15 ml Ethanol + 4.5 *µ*l Ultravist 300 ( Röntgenkontrastmittel der Schering AG, Berlin, Deutschland) + 1.35 ml Aceton + 0.8 mg Sudanrot + 30.0 mg Paclitaxel: Die gefalteten Ballonabschnitte der Katheter werden 5 x getaucht, das 1. Mal für 1 Minute, dann 3 h Trocknungszeit, danach 4 mal für je 5 sec im Abstand von 1 h; danach wurde ein Stent aufgecrimpt und der Katheter mit Stent in üblicher Weise mit Ethylenoxid sterilisiert: Paclitaxelgehalt 172 *µ*g, keine mittels HPLC detektierbaren Zersetzungsprodukte des Wirkstoffs
   b) Statt Ultravist 300 wird eine gesättigte wässrige Mannit-Lösung zugesetzt
   c) Statt Ultravist 300 wird eine gesättigte wässrige Natrium-Salicylat Lösung, pH 7.5, zugesetzt.
   d) Der fertigen Lösung nach (5a) werden 5 mg Acetylsalicylsäure zugesetzt.
   e) Der fertigen Lösung nach (5a) werden 5 mg Glycerin zugesetzt

### Vergleichsbeispiel 5:

Haftung des Wirkstoffs in Blut Es wurden 12 Ballonkatheter der Fa. BMT mit der Bezeichnung Allegro, Ballongröße 2.5 x 20 mm, verwendet. Je 6 Stück der gefalteten Ballonabschnitte der Katheter wurden entweder in [0.15 ml Ethanol + 4.5 *µ*l Ultravist 300 + 1.35 ml Aceton + 0.8 mg Sudanrot+ 30.0 mg Paclitaxel] oder in [1.5 ml Ethylacetat + 0.8 mg Sudanrot + 31.0 mg Paclitaxel] 5 x getaucht, das 1. Mal für 1 Minute, dann 3 h Trocknungszeit, danach 4 mal für je 5 sec im Abstand von 1 h; danach wurden je 3 gefaltete Ballone jeder Serie in 50 ml Humanblut 5 min bei 37 °C leicht bewegt und dann zur Analyse des Paclitaxel-Gehaltes entnommen: Verminderung der Mittelwerte (n=3 je Beschichtungsmethode) durch 5 Minuten Bewegung in Blut im Vergleich zu je 3 Kontrollkathetern, die nicht in Blut inkubiert werden.

| | |
|---|---|
| Aceton: | 12 % |
| Ethylacetat: | 10 % |

### Vergleichsbeispiel 6:

Untersuchung der Restenosehemmung nach Angioplastie und Stentimplantation an den Koronarien von Schweinen Gefaltete Ballonkatheter der Fa. BMT Typ Joker Lite, BMT 3.5 x 20 mm oder 3.0 x 20 mm wurden entweder in Lösung A) 3.33 ml Ethylacetat (EA) + 100.0 mg Paclitaxel oder in Lösung B) 0.45 ml Ethanol + 100 *µ*l Ultravist-370 + 4.5 ml Aceton (Ac) + 150.0 mg Paclitaxel für 1 min eingetaucht und bei Raurntemperatur über Nacht getrocknet. Ein weiterer (niedrige Dosis = L) bzw. 4 weitere (hohe Dosis = H) Tauchvorgänge wurden jeweils nur für 5 Sekunden am nächsten Tag im Abstand von 1 h durchgeführt.

Wirkstoffgehalt nach 2 maligem Tauchen in Lösung (B) im Mittel 250 *µ*g, bei 5 maligem Tauchen in Lösung (B) 500*µ*g, in Lösung (A) 400 *µ*g.

Insgesamt 22 Schweinen wurde mittels der mit Paclitaxel beschichteten Katheter oder mittels unbeschichteter Katheter Stents in die linke Vorderwand- oder Seitenwand-Koronararterie implantiert, wobei die Gefäße zur Stimulation der Restenose durch Gewebehyperplasie leicht überdehnt wurden. Nach 5 Wochen wurden die Tiere reangiographiert und das Maß der Gefäßverengung auf den Angiogrammen mit einem automatischen Computerprogramm gemessen.

| Gruppe | Stenose (%) |
|---|---|
| Unbeschichtet | 50.49 |
| AcL | 20.22 |
| EAH | 36.01 |
| AcH | 0.86 |
| p | .004 |

Quantitative Koronarangiographie 5 Wochen nach Stentimplantation mit unbeschichteten und beschichteten Kathetern; Stenose = prozentuale Verminderung des Lumendurchmessers im Bereich des Stents im Vergleich zu dem Lumendurchmesser unmittelbar nach Stentimplantation Mittelwert und statistische Signifikanz des Behandlungseffekts.

### Vergleichsbeispiel 7 :

Wirkstoffgehalt der Katheter nach Gefäßdilatation und Stentimplantation
Die Ballone aus Vergleichsbeispiel 7 wurden nach Stentimplantation und Entnahme aus den Tieren auf ca. 3 cm Länge von den Ballonkathetern abgetrennt und in 1.5 ml Ethanol überführt. Der Paclitaxelgehalt wurde mittels HPLC bestimmt. Alle verfügbaren beschichteten Ballone und eine Auswahl unbeschichteter Ballone wurden untersucht. Koronar,

| | | | |
|---|---|---|---|
| 3.0 x 20 mm, Beschichtung: | Ac hoch | 38 ± 4 *µ*g | (n=4) |
| | Ac niedrig | 22 ± 5 *µ*g | (n=2) |
| | EEE hoch | 41 | (n=1) |
| 3.5 x 20 mm, Beschichtung: | Ac hoch | 37 ±10 *µ*g | (n=8) |
| | Ac niedrig | 26 ± 6 *µ*g | (n=8) |
| | EEE hoch | 53 ± *µ*g | (n=9) |
| Unbeschichtet (unabhängig von Größe und Gefäßgebiet) | | | |
| | | 0.9 ± 1.0 *µ*g | (n=7) |

Aus Vergleichsbeispiel 5 ergibt sich, dass maximal 10 % der Dosis verloren gehen bevor der Ballon expandiert wird und ca. 10 % der Dosis auf dem Ballon verbleiben.

### Vergleichsbeispiel 8:

Probucol wird in einer Konzentration von 100 mg/ ml in Aceton eingebracht; die Lösung wird wie in den vorhergehenden Beispielen beschrieben zum Beschichten der Ballonkatheter eingesetzt.

### Vergleichsbeispiel 9:

Rapamycin wird in einer Konzentration von 10 mg/ ml in Diethylether gelöst. Die Beschichtung der Ballonanteile der Katheter erfolgt wie in den vorhergehenden Beispielen beschrieben; die Ballone sollen nach der Entnahme aus der Beschichtungslösung möglichst sofort horizontal ausgerichtet und ständig um ihre Längsachse gedreht werden.

### Vergleichsbeispiel 10:

Epothilon B wird in einer Konzentration von 2 mg/ ml in Ethylacetat gelöst; die Lösung wird wie in den vorhergehenden Beispielen beschrieben zum Beschichten der Ballonkatheter eingesetzt.

## Patentansprüche

1. Ballonkatheter zur Arzneimittelabgabe umfassend einen Ballon, wobei die Oberfläche des Ballons eine Beschichtung aufweist, **dadurch gekennzeichnet, dass** die Beschichtung einen stark lipophilen, schlecht wasserlöslichen, an beliebige Gewebebestandteile bindenden Wirkstoff und einen Zuckeralkohol umfasst, wobei der Wirkstoff ausgewählt ist aus der Gruppe umfassend Paclitaxel und andere Taxane, Rapamycin, Tacrolimus, Corticoide, Sexualhormone, Statine, Epothilone, Probucol, Prostacycline und Angiogeneseinduktoren, wobei der Zuckeralkohol ein Molekulargewicht < 2000 D aufweist und wobei der Wirkstoff in den Zuckeralkohol eingebettet ist.

2. Ballonkatheter nach Anspruch 1, erhältlich durch Auftragen einer Lösung bestehend aus dem Wirkstoff, dem Zuckeralkohol, und einem Lösungsmittel, welches ausgewählt ist aus Ethanol, Isopropanol, Ethylacetat, Diethylether, Aceton, Dimethylsulfoxid, Dimethylformamid, Glycerin und/oder Wasser, und Verdampfen des Lösungsmittels.

3. Ballonkatheter nach Anspruch 1, erhältlich durch Auftragen einer Lösung bestehend aus Paclitaxel, Mannit, Sudanrot und einem Lösungsmittel bestehend aus Ethanol, Aceton und Wasser, und Verdampfen des Lösungsmittels.

4. Ballonkatheter nach einem der vorherigen Ansprüche, **dadurch gekennzeichnet, dass** an der Oberfläche des Ballons der Wirkstoff in einer Dosierung von bis zu 5 µg/mm² vorliegt.

5. Ballonkatheter nach einem der vorherigen Ansprüche, **dadurch gekennzeichnet, dass** die Ballonoberfläche glatt, aufgeraut oder strukturiert ist.

6. Ballonkatheter nach einem der vorherigen Ansprüche, **dadurch gekennzeichnet, dass** das Kathetermaterial aus Polyamiden, Polyamid-Gemischen und Copolymeren, Polyethylenterephthalat, Polyethylen und Copolymeren, Polyurethan und Naturkautschuk ausgewählt ist.

7. Ballonkatheter nach einem der vorherigen Ansprüche, **dadurch gekennzeichnet, dass** der Ballon einen Durchmesser von 2-4 mm und eine Länge von 1 - 4 cm aufweist.

8. Ballonkatheter nach einem der vorherigen Ansprüche, wobei der Ballonkatheter in Verbindung mit einem Stent vorliegt.

9. Ballonkatheter nach einem der vorherigen Ansprüche, wobei der Ballonkatheter eine medizinische Vorrichtung zur Arzneimittelabgabe für die selektive Therapie bestimmter erkrankter Gewebeabschnitte oder Organteile ist, wobei an der Oberfläche des zumindest kurzzeitig das erkrankte Gewebe unter Druck kontaktierenden Ballons der lipophile, weitgehend wasserunlösliche an beliebige Gewebebestandteile bindende Wirkstoff mit nach Gewebekontakt sofortiger Wirkstofffreigabe haftet.

10. Verfahren zur Beschichtung eines Ballankatheters zur Arzneimittelabgabe, umfassend die Schritte:
a) Bereitstellen eines Ballonkatheters umfassend einen Ballon;
b) Auftragen einer Lösung bestehend aus einem stark lipophilen, schlecht wasserlöslichen, an beliebige Gewebebestandteile bindenden Wirkstoff, einem Zuckeralkohol, und einem Lösungsmittel auf die Oberfläche des Ballons, wobei der Zuckeralkohol ein Molekulargewicht < 2000 D aufweist, und wobei der Wirkstoff ausgewählt ist aus der Gruppe umfassend Paclitaxel und anderen Taxane, Rapamycin, Tacrolimus, Corticoide, Sexualhormone, Statine, Epothilone, Probucol, Prostacycline und Angiogeneseinduktoren;
c) Trocknen des Ballonkatheters.

11. Verfahren gemäß Anspruch 10, wobei das Auftragen durch Tauchen, Bestreichen, Auftragen mittels einer Volumenmesseinrichtung oder Besprühen erfolgt.

12. Verfahren gemäß Anspruch 10 oder 11, wobei das Lösungsmittel ausgewählt ist aus Ethanol, Isopropanol, Ethylacetat, Diethylether, Aceton, Dimethylsulfoxid, Dimethylformamid, Glycerin und/oder Wasser.

13. Verfahren nach einem der Ansprüche 10 bis 12, wobei Schritt (a) das Bereitstellen eines Ballonkatheters mit einem Ballon im gefalteten Zustand oder mit einem Ballon im entfalteten Zustand umfasst.

14. Verfahren nach einem der Ansprüche 10 bis 13, wobei Schritt (b) das Auftragen einer Lösung bestehend aus Paclitaxel, Mannit, Sudanrot und einem Lösungsmittel bestehend aus Aceton, Ethanol und Wasser umfasst.

15. Verwendung einer Lösung bestehend aus einem stark lipophilen, schlecht wasserlöslichen, an beliebige Gewebebestandteile bindenden Wirkstoff, einem Zuckeralkohol, und einem Lösungsmittel zur Beschichtung des Ballons eines Ballonkatheters zur Arzneimitteiabgabe, wobei der Zuckeralkohol ein Molekulargewicht < 2000 D aufweist und wobei der Wirkstoff ausgewählt ist aus der Gruppe umfassend Paclitaxel und anderen Taxane, Rapamycin, Tacrolimus, Corticoide, Sexualhormone, Statine, Epothilone, Probucol, Prostacycline und Angiogeneseinduktoren.

## Claims

1. Balloon catheter for delivery of medication comprising a balloon, wherein the surface of the balloon has a coating, **characterized in that** the coating comprises a strongly lipophilic, poorly water soluble drug that binds to any tissue parts; and a sugar alcohol, wherein the drug is selected from the group comprising paclitaxel and other taxanes, rapamycin, tacrolimus, corticoids, sexual hormones, statins, epothilones, probucol, prostacyclines and angiogenesis inducers, wherein the sugar alcohol has a molecular weight of < 2000 D and wherein the drug is embedded in the sugar alcohol.

2. Balloon catheter according to claim 1, obtainable by applying of a solution consisting of the drug, the sugar alcohol, and a solvent, wherein the solvent is selected from ethanol, isopropanol, ethyl acetate, diethyl ether, acetone, dimethyl sulfoxide, dimethyl formamide, glycerine, and/or water, and vaporizing the solvent.

3. Balloon catheter according to claim 1, obtainable by applying of a solution consisting of paclitaxel, mannitol, sudan red, and a solvent consisting of ethanol, acetone and water, and vaporizing the solvent.

4. Balloon catheter according to any of the preceding claims, **characterized in that** the drug is present on the surface of the balloon in a dosage of up to 5 µg/mm².

5. Balloon catheter according to any of the preceding claims, **characterized in that** the surface of the balloon is smooth, roughened or structured.

6. Balloon catheter according to any of the preceding claims, **characterized in that** the catheter material is selected from polyamides, polyamide-blends and copolymers, polyethylenterephthalate, polyethylene and copolymers, polyurethane, and natural rubber.

7. Balloon catheter according to any of the preceding claims, **characterized in that** the balloon has a diameter of 2-4 mm and a length of 1-4 cm.

8. Balloon catheter according to any of the preceding claims, wherein the balloon catheter is present in conjunction with a stent.

9. Balloon catheter according to any of the preceding claims, wherein the balloon catheter is a medical device for drug medication for the selective therapy of particular diseased tissue sections or organ parts, wherein the lipophilic, largely water insoluble drug that binds to any tissue parts adheres to the surface of the balloon, that contacts diseased tissue at least shortly under pressure, with immediate release of the drug upon tissue contact.

10. Process for coating a balloon catheter for delivery of medication, comprising the steps:
a) providing a balloon catheter comprising a balloon;
b) applying a solution consisting of a strongly lipophilic, poorly water soluble drug that binds to any tissue parts, a sugar alcohol, and a solvent to the surface of the balloon, wherein the sugar alcohol has a molecular weight of < 2000 D, and wherein the drug is selected from the group comprising paclitaxel and other taxanes, rapamycin, tacrolimus, corticoids, sexual hormones, statins, epothilones, probucol, prostacyclines and angiogenesis inducers;
c) drying the balloon catheter.

11. Process according to claim 10, wherein applying is conducted by dipping, painting, applying with a volume measuring device or by spraying.

12. Process according to claim 10 or 11, wherein the solvent is selected from ethanol, isopropanol, ethyl acetate, diethyl ether, acetone, dimethyl sulfoxide, dimethyl formamide, glycerine and/or water.

13. Process according to any of claims 10 to 12, wherein step (a) comprises providing a balloon catheter with a balloon in the folded state or with a balloon in the unfolded state.

14. Process according to any of claims 10 to 13, wherein step (b) comprises applying a solution consisting of paclitaxel, mannitol, sudan red, and a solvent consisting of acetone, ethanol and water.

15. Use of a solution consisting of a strongly lipophilic, poorly water soluble drug that binds to any tissue parts, a sugar alcohol, and a solvent for coating a balloon of a balloon catheter for delivery of medication, wherein the sugar alcohol has a molecular weight of < 2000 D and wherein the drug is selected from the group comprising paclitaxel and other taxanes, rapamycin, tacrolimus, corticoids, sexual hormones, statins, epothilones, probucol, prostacyclines and angiogenesis inducers.

## Revendications

1. Cathéter à ballonnet destiné à la délivrance de médicaments, comprenant un ballonnet, la surface du ballonnet étant pourvue d'un revêtement, **caractérisé en ce que** le revêtement comprend une substance active fortement lipophile peu soluble dans l'eau qui se lie à n'importe quel constituant tissulaire et un alditol, dans lequel la substance active est choisie dans le groupe comprenant le paclitaxel et autres taxanes, la rapamycine, le tacrolimus, les corticoïdes, les hormones sexuelles, les statines, les épothilones, le probucol, les prostacyclines et les inducteurs d'angiogenèse, dans lequel l'alditol possède une masse moléculaire inférieure à 2000 D et dans lequel la substance active est incorporée dans l'alditol.

2. Cathéter à ballonnet selon la revendication 1, susceptible d'être obtenu en déposant une solution constituée de la substance active, de l'alditol et d'un solvant choisi parmi l'éthanol, l'isopropanol, l'acétate d'éthyle, le diéthyléther, l'acétone, le diméthylsulfoxyde, le diméthylformamide, le glycérol et/ou l'eau, et en évaporant le solvant.

3. Cathéter à ballonnet selon la revendication 1, susceptible d'être obtenu en déposant une solution constituée de paclitaxel, de mannitol, de rouge soudan et d'un solvant constitué d'éthanol, d'acétone et d'eau, et en évaporant le solvant.

4. Cathéter à ballonnet selon l'une des revendications précédentes, **caractérisé en ce que** la substance active est présente sur la surface du ballonnet à une dose allant jusqu'à 5 µg/mm².

5. Cathéter à ballonnet selon l'une des revendications précédentes, **caractérisé en ce que** la surface du ballonnet est lisse, rendue rugueuse ou structurée.

6. Cathéter à ballonnet selon l'une des revendications précédentes, **caractérisé en ce que** le matériau du cathéter est choisi parmi les polyamides, les mélanges et copolymères à base de polyamide, le polytéréphtalate d'éthylène, le polyéthylène et ses copolymères, le polyuréthane et le caoutchouc naturel.

7. Cathéter à ballonnet selon l'une des revendications précédentes, **caractérisé en ce que** le ballonnet possède un diamètre de 2 à 4 mm et une longueur de 1 à 4 cm.

8. Cathéter à ballonnet selon l'une des revendications précédentes, dans lequel le cathéter à ballonnet est présent en association avec un stent.

9. Cathéter à ballonnet selon l'une des revendications précédentes, dans lequel le cathéter à ballonnet est un dispositif médical, destiné à la délivrance de médicaments en vue du traitement sélectif de certaines portions tissulaires ou parties d'organes malades, dans lequel la substance active lipophile pratiquement insoluble dans l'eau qui se lie à n'importe quel constituant tissulaire adhère à la surface du ballonnet au moins brièvement en contact sous pression avec le tissu malade, avec libération de la substance active immédiatement après le contact avec le tissu.

10. Procédé de revêtement d'un cathéter à ballonnets destiné à la délivrance de médicaments, comprenant les étapes suivantes :
a) fourniture d'un cathéter à ballonnet comprenant un ballonnet ;
b) dépôt d'une solution constituée d'une substance active fortement lipophile peu soluble dans l'eau qui se lie à n'importe quel constituant tissulaire, d'un alditol et d'un solvant sur la surface du ballonnet, dans lequel alditol possède une masse moléculaire inférieure à 2000D et dans lequel la substance active est choisie dans le groupe comprenant le paclitaxel et autres taxanes, la rapamycine, le tacrolimus, les corticoïdes, les hormones sexuelles, les statines, les épothilones, le probucol, les prostacyclines et les inducteurs d'angiogenèse ;
c) séchage du cathéter à ballonnet.

11. Procédé selon la revendication 10, dans lequel le dépôt se fait par trempage, enduction, application au moyen d'un dispositif volumétrique ou pulvérisation.

12. Procédé selon la revendication 10 ou la revendication 11, dans lequel le solvant est choisi parmi l'éthanol, l'isopropanol, l'acétate d'éthyle, le diéthyléther, l'acétone, le diméthylsulfoxyde, le diméthylformamide, le glycérol et/ou l'eau.

13. Procédé selon l'une des revendications 10 à 12, dans lequel l'étape (a) comprend l'obtention d'un cathéter à ballonnet pourvu d'un ballonnet à l'état replié ou d'un ballonnet à l'état déplié.

14. Procédé selon l'une des revendications 10 à 13, dans lequel l'étape (b) comprend le dépôt d'une solution constituée de paclitaxel, de mannitol, de rouge soudan et d'un solvant constitué d'acétone, d'éthanol et d'eau.

15. Utilisation d'une solution constituée d'une substance active fortement lipophile peu soluble dans l'eau qui se lie à n'importe quel constituant tissulaire, d'un alditol et d'un solvant pour revêtir le ballonnet d'un cathéter à ballonnet destiné à la délivrance de médicaments, dans lequel l'alditol possède une masse moléculaire inférieure à 2000 D et dans lequel la substance active est choisie dans le groupe comprenant le paclitaxel et autres taxanes, la rapamycine, le tacrolimus, les corticoïdes, les hormones sexuelles, les statines, les épothilones, le probucol, les prostacyclines et les inducteurs d'angiogenèse.
